# EUROPEAN PATENT APPLICATION

(11) **EP 2 196 804 A1**
(43) Date of publication of application: **16.06.2010**
(21) Application number: 08835186.1
(22) Date of filing: 24.09.2008
(51) Int. Cl.: G01N 33/532, G01N 33/53, C07K 14/31, C07K 14/315, C07K 14/47

(54) **LABELED PEPTIDE HAVING ACTIVITY OF BINDING TO IMMUNOGLOBULIN AND/OR IMMUNOGLOBULIN COMPLEX AND METHOD OF DETECTING OR ASSAYING IMMUNOGLOBULIN BY USING THE PEPTIDE**

(30) Priority: 05.10.2007 JP 2007262110
(71) Applicant: Kaneka Corporation, Kita-ku Osaka-shi, Osaka 530-8288 (JP)
(72) Inventor: SATO, Nobuhiko, c/o Kaneka Corporation, Settsu-shi, Osaka 566-0072 (JP); OHARA, Kazumasa, c/o Kaneka Corporation, Settsu-shi, Osaka 566-0072 (JP); OGINO, Eiji, c/o Kaneka Corporation, Settsu-shi, Osaka 566-0072 (JP)
(74) Representative: Vossius & Partner
(86) International application number: PCT/JP2008/067171
(87) International publication number: WO 2009/044650

(57) **Abstract**

Disclosed is (i) a compound which makes it possible to economically and easily detect or assay immunoglobulins under the same conditions regardless of animal species and (ii) a method for detecting or assaying an immunoglobulin through use of the compound.

Use of a peptide carrying a labeling agent and having activity of binding to an immunoglobulin and/or an immunoglobulin complex makes it possible to economically and easily detect or assay immunoglobulins under the same conditions regardless of animal species.

SEQUENCE LISTING

<110>  KANEKA CORPORATION

<120>  LABELED PEPTIDE HAVING ACTIVITY OF BINDING TO IMMUNOGLOBULIN AND/OR
IMMUNOGLOBULIN COMPLEX AND METHOD OF DETECTING OR ASSAYING IMMUNOGLOBULIN BY
USING THE PEPTIDE

<130>  S1540 EP

<140>  EP 08 83 5186.1
<141>  2008-09-24

<150>  JP 2007-262110
<151>  2007-10-05

<160>  3

<170>  PatentIn version 3.1

<210>  1
<211>  58
<212>  PRT
<213>  Artificial sequence

<220>
<223>  /note="Description of artificial sequence: PGMG"

<400>  1

Met Thr Thr Tyr Lys Leu Val Ile Asn Gly Lys Thr Leu Lys Gly Glu
1               5                   10                  15

Thr Thr Thr Lys Ala Val Asp Ala Glu Thr Ala Glu Arg Ala Phe Arg
            20                  25                  30

Gln Tyr Ala Thr Asp Asn Gly Val Glu Gly Met Trp Thr Tyr Asp Pro
        35                  40                  45

Ala Thr Lys Thr Phe Thr Val Thr Glu Cys
    50                  55

<210>  2
<211>  13
<212>  PRT
<213>  Artificial sequence

<220>
<223>  /note="Description of artificial sequence: beta-1-adrenoceptor loop 1
antigen"

<220>
<221>  MOD_RES
<222>  (1)..(1)
<223>  ACETYLATION

<400>  2

Glu Tyr Gly Ser Phe Phe Cys Glu Leu Trp Thr Ser Cys
1               5                   10

<210>  3

<211>  27
<212>  PRT
<213>  Artificial sequence

<220>
<223>  /note="Description of artificial sequence: beta-1-adrenoceptor loop 2
antigen"

<400>  3

His Trp Trp Arg Ala Glu Ser Asp Glu Ala Arg Arg Cys Tyr Asn Asp
1               5                   10                  15

Pro Lys Cys Cys Asp Phe Val Thr Asn Arg Cys
            20                  25

## Description

### Technical Field

The present invention relates to providing of a peptide which makes it possible to economically and easily detect or assay immunoglobulins in samples under the same conditions regardless of animal species. The present invention also relates to: a method for detecting or assaying an immunoglobulin through use of the peptide; and an immunoglobulin detection or assay kit using the peptide.

### Background Art

After entering into the 21st century, completion of the decoding of human genome was announced. In response to this, efforts to develop a wide variety of drugs, including immunoglobulin drugs (antibody drugs), have been made vigorously. Most of the heretofore developed immunoglobulin drugs are of IgG antibodies, which belong to immunoglobulin G. The IgG antibody is constituted by (i) the Fab region, which is a variable region specifically recognizing an antigen, and (ii) the Fc region, which is a constant region. Taking advantage of the diversity of the variable region of the IgG antibody, the immunoglobulin drug binds to only a certain substance specifically so as to inhibit physiological activity or, on the contrary, to enhance activity. Therefore, the immunoglobulin drug gives less side effects.

Development of an immunoglobulin drug starts with an immunoglobulin including the Fab region and the Fc region both derived from an animal (e.g., a mouse or a rat) which is not a human, in consideration of convenience in e.g., screening using an antigen. However, use of a fully different-species animal immunoglobulin, constituted by regions all derived from an animal species which is not a human, may cause a big problem mainly in a safety aspect. For example, in a case where the fully different-species animal immunoglobulin is administered to a human, the fully different-species animal immunoglobulin is recognized as foreign, thereby leading to production of an immunoglobulin against the fully different-species animal immunoglobulin. In order to avoid this, the fully different-species animal immunoglobulin is converted into a so-called chimeric immunoglobulin. In the chimeric immunoglobulin, the Fab region recognizing an antigen is still derived from the animal of the different species, whereas the Fc region, which is the constant region, is modified to one derived from a human. If this does not still overcome the problem of safety, the chimeric immunoglobulin is converted into a humanized immunoglobulin. In the humanized immunoglobulin, of the Fab region recognizing an antigen, only a narrower region recognizing the antigen, i.e., a complementarity-determining region is derived from the animal of the different species, whereas the other regions of the Fab region and the Fc region are humanized. Ultimately, the immunoglobulin can be converted into a fully human immunoglobulin, which has been humanized completely. Thus, in the case where the fully different-species animal immunoglobulin is converted into the chimeric immunoglobulin, the chimeric immunoglobulin into the humanized immunoglobulin, and the humanized immunoglobulin into the fully human immunoglobulin, their binding forces vary even with respect to the same antigen. However, it is difficult to make comparison between their binding properties against the antigen under the same conditions, because these immunoglobulins are derived from animals of different species or a combination of animals of different species. What is more, bioassays using animals or cells are troublesome, and it is very difficult to determine quantity by the bioassays. Therefore, it is impossible to assay, under the same conditions, binding properties and activity of immunoglobulins derived from animals of different species. Furthermore, it is difficult to evaluate, with an absolute indication parameter, the binding forces of these immunoglobulins.

Further, also in terms of diagnosis of specified diseases and researches on intractable diseases, a basic technology for assaying a wide variety of immunoglobulins under the same conditions and with the same standard is required. Generally, for a research on a certain disease or on how to treat it (e.g., a drug), a model animal for the disease of interest is produced to perform an experiment. For example, a model animal used for a research on an autoimmune disease is the one having a specific immunoglobulin induced therein by immunization with an antigen on which an autoimmune globulin acts. From the model animal thus produced, basic data regarding a binding property and inhibition activity of the autoimmune globulin is obtained and accumulated; however, there is a serious hindrance in applying the basic data to a human. Even if the immunoglobulin of the model animal and an immunoglobulin of a human are the same, they are different from each other in terms of the binding properties and activity against the antigen, since they are derived from animals of different species. Therefore, it is impossible to assay these immunoglobulins under the same conditions and with the same standard. This leads to a big problem that it is impossible to find the correspondence between these immunoglobulins and to make comparison between these immunoglobulins.

In addition, in quantitative determination of a specific immunoglobulin in a body fluid for diagnosis of a specified disease, it is impossible to evaluate an antibody titer and activity of the specific immunoglobulin in the body fluid by replacing the antibody titer and the activity with an absolute indication parameter, because there is no means for evaluating, under the same conditions, a test sample and an immunoglobulin of a different animal species, which serves as a standard indication (Non-Patent Literature 1 and Non-Patent Literature 2).

This, however, is enabled by the following compounds: fluorescence-labeled Protein A, fluorescence-labeled Protein G, or the like. However, these proteins have many problems. For example, these proteins, each having a molecular mass of several tens of thousands of daltons, are easily deactivated, difficult to be handled, and expensive.

For the foregoing reasons, there is a need for: a compound making it possible to economically and easily detect or assay, under the same conditions, a wide variety of immunoglobulins in samples regardless of animal species; or a method for detecting or assaying an immunoglobulin through use of the compound.
Non-Patent Literature 1
   Kaisa Granfors, Matti K. Viljanen, and Auli Toivanen, Journal of Clinical Microbiology, Vol. 14, No. 1, July 1981, p.6-14
Non-Patent Literature 2
   Mark P. Hedger And Shyamani Hettiarachchi, Journal of Andrology, Vol. 15, No. 6, November/December 1994, p.583-590

### Summary of Invention

### Technical Problem

An object of the present invention is to provide: a compound making it possible to economically and easily detect or assay immunoglobulins in samples under the same conditions regardless of animal species; a method for detecting or assaying an immunoglobulin through use of the compound; and an immunoglobulin detection or assay kit using the compound.

### Solution to Problem

The inventors of the present invention made a diligent study on a method for economically and easily detecting or assaying immunoglobulins in samples under the same conditions regardless of animal species. As a result, the inventors of the present invention found the following fact: Use of a labeled peptide having activity of binding to an immunoglobulin and/or an immunoglobulin complex makes it possible to detect or assay an immunoglobulin in a sample economically and easily and regardless of animal species from which the immunoglobulin derived. Thus, the present invention was completed. By using the detecting method or the assaying method of the present invention, it is possible to assay an antibody titer of a human and an antibody titer of a different animal at once. Therefore, in a case where an antibody titer of a certain animal is set as a standard, it is possible to calculate an antibody titer of a human based on the standard antibody titer, serving as an indication, and to compare, with the same standard, the antibody titer of the human with an antibody titer of an animal species which is not the standard animal.

That is, the present invention relates to a method for economically and easily detecting or assaying an immunoglobulin regardless of animal species.

A peptide of the present invention having activity of binding to an immunoglobulin and/or an immunoglobulin complex is at least one selected from: a partial peptide of Protein A; a partial peptide of Protein G; a partial peptide of Protein H; a partial peptide of Protein M; a partial peptide of a rheumatoid factor; and a partial peptide of a complement. The peptide may be a peptide obtained by mutation of the partial peptide of Protein A, Protein G, Protein H, Protein M, the rheumatoid factor or the complement.

Here, the peptide obtained by mutation of the partial peptide of Protein A, Protein G, Protein H, Protein M, the rheumatoid factor or the complement, i.e., a mutant of the partial peptide of Protein A, Protein G, Protein H, Protein M, the rheumatoid factor or the complement is one having an amino acid sequence with a substitution, deletion, insertion, and/or addition of one or several amino acids in an amino acid sequence of the partial peptide of Protein A, Protein G, Protein H, Protein M, the rheumatoid factor or the complement, the one having activity of binding to an immunoglobulin. Note that the above expression "an amino acid sequence with a substitution, deletion, insertion, and/or addition of one or several amino acids in an amino acid sequence" means a substitution, deletion, insertion, and/or addition of numbers of amino acids (preferably 10 or less, more preferably 7 or less, most preferably 5 or less) that can be optionally brought about by a known mutant polypeptide producing method. Such a mutant polypeptide is not limited to a polypeptide made by artificial introduction of mutation by means of a known mutant polypeptide producing method, but may be one obtained by isolation and purification of a naturally-existing polypeptide. The mutation is preferably a conservative or nonconservative substitution, deletion, and/or addition of an amino acid(s), more preferably a silent substitution, addition, and/or deletion thereof, and particularly preferably a conservative substitution thereof.

A labeling agent for the peptide having activity of binding to an immunoglobulin and/or an immunoglobulin complex is, to be specific, a radioactive substance, an enzyme, a coenzyme, a luminescence substance, a fluorescence substance, or a fluorescent protein. The labeling agent may be contained in the peptide having activity of binding to an immunoglobulin and/or an immunoglobulin complex or bonded to the peptide through covalent bonding.

### Advantageous Effects of Invention

A method of the present invention for detecting or assaying an immunoglobulin through use of a labeled peptide having activity of binding to an immunoglobulin and/or an immunoglobulin complex is an economical and easy method. Further, the labeled peptide having activity of binding to an immunoglobulin and/or an immunoglobulin complex has activity of binding to a wide variety of immunoglobulins regardless of animal species; therefore, it is possible to assay or detect immunoglobulins of a wide variety of animal species by the same method. Thus, the detection method and the assay method of the present invention are clearly different from conventional methods, and are expected to be applied to a wide range of fields including the medical field, for example, for assaying pathogenic substances and disease markers and for investigating etiologies of intractable diseases.

Additional objects, features, and strengths of the present invention will be made clear by the description below.

### Description of Embodiments

A peptide of the present invention having activity of binding to an immunoglobulin and/or an immunoglobulin complex is, specifically, a partial peptide of Protein A, a partial peptide of Protein G, a partial peptide of Protein H, a partial peptide of Protein M, a partial peptide of a rheumatoid factor or a partial peptide of a complement. The peptide of the present invention may be a mutant of the partial peptide of Protein A, Protein G, Protein H, Protein M, the rheumatoid factor or the complement. The peptide of the present invention is preferably the partial peptide of Protein G or the mutant of the partial peptide. The above peptides, each having activity of binding to an immunoglobulin and/or an immunoglobulin complex, may solely be used, or may be used in combination.

Further, it is preferable that the peptide of the present invention, having activity of binding to an immunoglobulin and/or an immunoglobulin complex, is capable of binding to immunoglobulins of not necessarily all of, but many of animal species such as a human, a mouse, a rat, a rabbit, a sheep, a pig, a horse, a dog, a goat, a hamster, a guinea pig, a cow, and a chicken. Furthermore, it is more preferable that the peptide of the present invention has activity of binding to classes and subclasses of immunoglobulins. Note that the above animal species are presented just as typical examples, and the present invention is not limited to these. Further, the peptide of the present invention, having activity of binding to an immunoglobulin and/or an immunoglobulin complex, is not specified as being capable of binding to immunoglobulins of all animal species.

A labeling agent of the present invention is a radioactive substance, an enzyme, a coenzyme, a luminescence substance, a fluorescence substance or a fluorescent protein. More specifically, the labeling agent of the present invention is 35S methionine, HRP (horseradish peroxidase), alkaline phosphatase, biotin, an acridinium derivative, fluorescein or GFP (Green Fluorescent Protein). These labeling agents may solely be used, or may be used in combination.

Typical examples of the radioactive substance serving as the labeling agent encompass an amino acid labeled with radioactive iodine, radioactive carbon, radioactive sulfur, radioactive hydrogen or radioactive phosphor.

Listed above as an example of the enzyme are: alkaline phosphatase, which reacts with a substrate so as to induce light emission, fluorescence emission, or coloring; and HRP, which induces light emission. In addition to these, typical examples of such the enzyme encompass: galactosidase, which generates fluorescence; and luciferase, which reacts with luciferin (a substrate) so as to emit fluorescence.

Typical examples of the fluorescence substance encompass Cydy, fluorescein, Alexa Fluor dye, MFP488, and rhodamin. Examples of the fluorescent protein encompass, in addition to GFP, REP (Red Fluorescent Protein), YFP (Yellow Fluorescent Protein), and CFP (Cyan Fluorescent Protein). The above labeling agents are presented just as typical examples, and the present invention is not particularly limited to these labeling agents.

Labeling is possible not only by the above-mentioned labeling agents but also by a peptide tag sequence introduced into or fused with the peptide having activity of binding to an immunoglobulin and/or an immunoglobulin complex. For example, an S tag sequence peptide has catalytic activity (ribonuclease S activity), that is, the S tag sequence peptide cleaves RNA once the S tag sequence peptide is bonded to S Protein. Therefore, addition of mRNA quenched by FRET to the S tag sequence peptide causes fluorescence emission. According to this known method (see Proc. Natl. Acad. Sci. USA Vol. 96, p2019-2024, 1999), it is possible to use the S tag sequence peptide as the labeling agent to detect or assay an immunoglobulin.

The labeled peptide of the present invention having activity of binding to an immunoglobulin and/or an immunoglobulin complex is obtained by various methods. Generally, a relatively short peptide is obtained by a solid-phase synthesis, whereas a relatively long peptide is synthesized through production by bacteria such as *E*. *coli.* Recently, a cell-free protein synthesis system (see The FEBS Journal Vol.273, p4133-4140, 2006) is also used to synthesize long and short peptides.

In the case where the peptide having activity of binding to an immunoglobulin and/or an immunoglobulin complex is short, a labeling agent of interest may be added as one of building blocks for solid-phase synthesis. The labeling agent used in this method is not particularly limited. It is considered that, by using an amino group and/or a thiol group contained in the peptide, any of all foregoing labeling agents may be added to or included in the peptide. Alternatively, of course, a labeling agent may be bonded to the peptide after solid-phase synthesis. Further alternatively, the labeled peptide can be prepared by bonding the target peptide to a labeling peptide or a labeling agent by means of a chemical ligation method (see J. Am. Chem. Soc., Vol.128, p6640-6646, 2006).

In the case where the peptide having activity of binding to an immunoglobulin and/or an immunoglobulin complex is relatively long, the peptide, produced by bacteria, may be labeled through an amino group and/or a thiol group with a radioactive substance, an enzyme, a coenzyme, a fluorescence substance, or a fluorescence protein.

In the case where the labeling agent is an enzyme, a peptide tag or a fluorescent protein, labeling may be made in such a manner that the labeling agent is subjected to genetic manipulation so as to be fused with the peptide having activity of binding to an immunoglobulin and/or an immunoglobulin complex. Further, in the case where the labeling agent is substantially the same as a natural amino acid (e.g., in the case of 35S methionine), labeling may be made in such a manner that the labeling agent is added to a culture solution instead of a natural amino acid.

In addition to the above methods, there is a method of labeling through a non-natural amino acid introduction method (see Nature Methods Vol.3, p923-929, 2006; Proc. Natl. Acad. Sci. USA Vol.103, p9785-9789, 2006). It has been confirmed that this method allows many side-chain modified amino acids such as fluorescence substances and radioactive substances to be introduced into proteins. Described herein are typical methods for producing the labeled peptide having activity of binding to an immunoglobulin and/or an immunoglobulin complex, and the present invention is not limited to these.

Specific examples of the method of the present invention for assaying or detecting an immunoglobulin encompass detection methods and assaying methods utilizing an immunoassay, e.g., ELISA (Enzyme-linked Immuno Sorbent Assay).

First specific example of this method is as follows: Firstly, a solution containing an immunoglobulin is brought into contact with a solid phase such as a plate or a well, so that the immunoglobulin and/or the immunoglobulin complex contained in the solution is bound to or adsorbed onto the solid phase. The solid phase is then washed, and is blocked by appropriate means. Subsequently, a labeled peptide having activity of binding to an immunoglobulin and/or an immunoglobulin complex is added thereto. Lastly, the solid phase is washed, and the peptide, having the activity of binding to an immunoglobulin and/or an immunoglobulin complex, which is specifically bound to the immunoglobulin on the solid phase is detected or assayed according to the labeling agents. Thus, the immunoglobulin in the solution is detected or assayed.

Second specific example of the above method may be a method for detecting or assaying an immunoglobulin having specificity for an antigen. Firstly, an antigen is directly or indirectly bound to or adsorbed onto a solid phase by appropriate means, for example, by causing an antigen conjugated to BSA to come into contact with a solid phase such as a plate or a well. The solid phase is blocked by appropriate means if necessary, and then a solution containing an immunoglobulin is added thereto, so that the immunoglobulin reactive with the antigen is specifically bound to the antigen. The solid phase is then washed, and a labeled peptide having activity of binding to an immunoglobulin and/or an immunoglobulin complex is added thereto, so that the labeled peptide is bound to the immunoglobulin specifically bound to the antigen. After washing, the specific immunoglobulin bound to the antigen is detected or assayed according to the labeling agent, in the same manner as in the first specific example.

The detection or the assay of an immunoglobulin using the labeling agent is made possible by direct or indirect detection of a signal given by the labeling agent. For example, with a compound giving a signal by itself (e.g., the above-described radioactive substance, fluorescence substance or fluorescent protein), it is possible to directly detect the signal with use of an appropriate device without any external effects (except for irradiation) or addition of another substance. On the other hand, enzymes such as HRP, alkaline phosphatase, galactosidase, and luciferase do not give a signal unless being subjected to an external stimulus. Therefore, it is necessary to add a suitable substrate for each of these enzymes under suitable conditions for each of these enzymes so that the enzyme is induced to give a signal such as light emission, fluorescence emission or coloring. In some cases, the signal is caused to stop. In the case where the labeling agent is any of the above enzymes, the labeling agent is induced to give a signal in this manner, and the signal is thus indirectly detected. The detection of the signal is performed similarly with use of an appropriate device. In the case where the labeling agent is biotin, avidin/streptoavidin to which biotin specifically binds is generally used. Avidin/streptoavidin having e.g., a fluorescent dye, a radioactive substance, HRP, alkaline phosphatase, galactosidase and/or luciferase added thereto or contained therein is used to induce signal release depending on an immunoglobulin.

The present invention is considered to be applicable to not only the detection or the assay of an immunoglobulin but also (i) identification of an antigen recognized by an immunoglobulin and (ii) epitope-mapping. For example, various antigens are immobilized onto a 96-well plate, and a solution containing an immunoglobulin is added thereto; then, a labeled peptide of the present invention having activity of binding to an immunoglobulin and/or an immunoglobulin complex is used, and a labeling signal is detected. In this way, the present invention can be applied to the identification of the antigen recognized by the immunoglobulin. Also, the epitope-mapping of an immunoglobulin may be performed in such a manner that a selected antigen is immobilized to a 96-well plate and the same procedure as above are performed. The method of the present invention, which works regardless of animal species, also enables a high-throughput antigen screening of immunoglobulins with the same kit. In addition, in study of an interaction between an immunoglobulin and a receptor expressed in a certain cell, the immunoglobulin bound to the cell can be detected by using the labeled peptide of the present invention having activity of binding to an immunoglobulin and/or an immunoglobulin complex.

The present invention is not limited to the description of the embodiments above, but may be altered by a skilled person within the scope of the claims. An embodiment based on a proper combination of technical means disclosed in different embodiments is encompassed in the technical scope of the present invention.

### Examples

In the following Examples, the present invention will be described in detail. However, the present invention is not limited to the Examples below.

### (Example 1)

### <Production of Labeled Partial Peptide of Protein G>

Maleimidized HRP (30 mg) was dissolved in a phosphate buffer. Next, a partial peptide (50 mg) derived from Protein G (PGMP) was dissolved in a phosphate buffer. This partial peptide was obtained through production by recombinant microorganisms, and had the amino acid sequence shown in SEQ ID NO: 1 (see the descriptions in Japanese Patent Application, Tokuganhei, No. 9-526738). The partial peptide solution was added to the HRP solution. Subsequently, the resultant solution was dialyzed and freeze-dried, so that an HRP-conjugated PGMP was given.

### <Immunoassay of Human Beta-1-Adrenoceptor Antibody to Beta-1-Adrenoceptor Loop 1 Antigen>

A beta-1-adrenoceptor Loop 1 antigen (bAR-L1) having the amino acid sequence shown in SEQ ID NO: 2 was conjugated to bovine serum albumin (BSA), so that BSA-L1 was given. The BSA-L1 thus given was dissolved in 0.05M carbonate buffer (pH 9.6), so that a solution was prepared at 3 µg/mL.

100 µL of the solution was added to each well of a 96-well plate (IMMUNO PLATE; available from NUNC), and the plate was left at rest at 37°C for an hour. Each well was washed with 300 µL of 0.05% Tween 20/PBS buffer (pH 7.2) five times. Thereafter, BSA was dissolved in a PBS buffer (pH 7.2) to give a blocking solution (1% BSA). 200 µL of the blocking solution was added to each well, and left at rest at 37°C for an hour for blocking.

Next, a polyclonal antibody, serving as a standard indication, was prepared by purification from a rabbit immunized with a Loop 1 antigen. The polyclonal antibody was diluted sequentially, so as to be used as a sample for establishment of a standard curve. The serum of a dilated cardiomyopathy patient was used as a sample. 100 µL of the sample for establishment of the standard curve was added to each of some wells of the plate having been blocked, and 100 µL of the suitably-diluted serum sample of the dilated cardiomyopathy patient was added to each of the other wells of the same plate. Then, the plate was left at rest at 37°C for an hour for an antigen-antibody reaction. Thereafter, each well was washed with 300 µL of 0.05% Tween 20/PBS buffer (pH 7.2) five times.

Lastly, PGMP (100 µg/mL) was conjugated with a labeling agent HRP dissolved in a physiological saline, and 100 µL of the resultant was added to each well, which was then left at rest for an hour. Then, each well was washed with 300 µL of 0.05% Tween 20/PBS buffer (pH 7.2) five times, and 100 µL of a solution containing OPD (ortho-phenylenediamine), which was a substrate of HRP, (a citric acid solution containing 0.0001% hydrogen peroxide and 0.1% OPD), was added to each well. The wells were left at rest at 37°C for 30 minutes, and then 50 µL of a 4N sulfate solution was added to each well so as to stop the reaction. Then, an absorbency was measured at a wavelength of 492 nm with a microplate reader. Thus, the immunoassay of the human beta-1-adrenoceptor antibody to the beta-1-adrenoceptor Loop 1 antigen was performed. The result is shown in Table 1.

### (Example 2)

### <Immunoassay of Human Beta-1-Adrenoceptor Antibody to Beta-1-Adrenoceptor Loop 2 Antigen>

A beta-1-adrenoceptor Loop 2 antigen (bAR-L2) having the amino acid sequence shown in SEQ ID NO: 3 was conjugated to bovine serum albumin (BSA), so that BSA-L2 was given. The BSA-L2 thus given was dissolved in 0.05M carbonate buffer (pH 9.6), so that a solution was prepared at 50 µg/mL. Thereafter, immobilization onto a plate and blocking were performed in the same manner as in Example 1.

A polyclonal antibody purified from a rabbit immunized with a Loop 2 antigen, serving as a standard indication, was diluted sequentially, so as to be used as a sample for establishment of a standard curve. Except for this, an immunoassay of the human beta-1-adrenoceptor antibody to the beta-1-adrenoceptor Loop 2 antigen was performed in the same manner as in Example 1. The result is shown in Table 1.

| [Table 1] | | |
|---|---|---|
| | Antibody Titer against Loop 1 | Antibody Titer against Loop 2 |
| Ex. 1 | 3.0 | - |
| Ex. 2 | - | 49 |

| | | |
|---|---|---|
| Unit: µg/mL Abbreviation: "Ex." stands for "Example". | | |

### (Example 3)

### <Immunoassay of Rabbit Beta-1-Adrenoceptor Antibody to Beta-1-Adrenoceptor Loop 1 Antigen>

A beta-1-adrenoceptor Loop 1 antigen (bAR-L1) having the amino acid sequence shown in SEQ ID NO: 2 was conjugated to bovine serum albumin (BSA), so that BSA-L1 was given. The BSA-L1 thus given was dissolved in 0.05M carbonate buffer (pH 9.6), so that a solution was prepared at 3 µg/mL. Thereafter, immobilization onto a plate and blocking were performed in the same manner as in Example 1.

A serum sample of a rabbit immunized with a Loop 1 antigen was used as a sample. Except for this, an immunoassay of the rabbit beta-1-adrenoceptor antibody to the beta-1-adrenoceptor Loop 1 antigen was performed in the same manner as in Example 1. The result is shown in Table 2.

### (Example 4)

### <Immunoassay of Rabbit Beta-1-Adrenoceptor Antibody to Beta-1-Adrenoceptor Loop 2 Antigen>

A beta-1-adrenoceptor Loop 2 antigen (bAR-L2) having the amino acid sequence shown in SEQ ID NO: 3 was conjugated to bovine serum albumin (BSA), so that BSA-L2 was given. The BSA-L2 thus given was dissolved in 0.05M carbonate buffer (pH 9.6), so that a solution was prepared at 10 µg/mL. Thereafter, immobilization onto a plate and blocking were performed in the same manner as in Example 2.

A serum sample of a rabbit immunized with a Loop 2 antigen was used as a sample. Except for this, an immunoassay of the rabbit beta-1-adrenoceptor antibody to the beta-1-adrenoceptor Loop 2 antigen was performed in the same manner as in Example 2. The result is shown in Table 2.

| [Table 2] | | |
|---|---|---|
| | Antibody Titer against Loop 1 | Antibody Titer against Loop 2 |
| Ex. 3 | 170 | - |
| Ex. 4 | - | 220 |

| | | |
|---|---|---|
| Unit: µg/mL Abbreviation: "Ex." stands for "Example". | | |

### (Example 5)

### <Immunoassay of Piglet Beta-1-Adrenoceptor Antibody to Beta-1-Adrenoceptor Loop 1 Antigen>

A beta-1-adrenoceptor Loop 1 antigen (bAR-L1) having the amino acid sequence shown in SEQ ID NO: 2 was conjugated to bovine serum albumin (BSA), so that BSA-L1 was given. The BSA-L1 thus given was dissolved in 0.05M carbonate buffer (pH 9.6), so that a solution was prepared at 3 µg/mL. Thereafter, immobilization onto a plate and blocking were performed in the same manner as in Example 1.

A serum sample of a piglet immunized with a Loop 1 antigen was used as a sample. Except for this, an immunoassay of the piglet beta-1-adrenoceptor antibody to the beta-1-adrenoceptor Loop 1 antigen was performed in the same manner as in Example 1. The result is shown in Table 3.

### (Example 6)

### <Immunoassay of Piglet Beta-1-Adrenoceptor Antibody to Beta-1-Adrenoceptor Loop 2 Antigen>

A beta-1-adrenoceptor Loop 2 antigen (bAR-L2) having the amino acid sequence shown in SEQ ID NO: 3 was conjugated to bovine serum albumin (BSA), so that BSA-L2 was given. The BSA-L2 thus given was dissolved in 0.05M carbonate buffer (pH 9.6), so that a solution was prepared at 50 µg/mL. Thereafter, immobilization onto a plate and blocking were performed in the same manner as in Example 2.

A serum sample of a piglet immunized with a Loop 2 antigen was used as a sample. Except for this, an immunoassay of the piglet beta-1-adrenoceptor antibody to the beta-1-adrenoceptor Loop 1 antigen was performed in the same manner as in Example 2. The result is shown in Table 3.

| [Table 3] | | |
|---|---|---|
| | Antibody Titer against Loop 1 | Antibody Titer against Loop 2 |
| Ex. 5 | 0.056 | - |
| Ex. 6 | - | 26.8 |

| | | |
|---|---|---|
| Unit: µg/mL Abbreviation: "Ex." stands for "Example". | | |

### Industrial Applicability

As described above, use of a peptide of the present invention or a method for detecting or assaying an immunoglobulin using the peptide makes it possible to economically and easily assay or detect immunoglobulins of a wide variety of animal species. Therefore, the present invention can be used to assay pathogenic substances and disease markers, and to investigate etiologies of intractable diseases. Thus, the present invention is applicable to a wide range of fields including the medical field and the pharmaceutical field.

## Claims

1. A peptide comprising or being bonded with a labeling agent, the peptide having activity of binding to an immunoglobulin and/or an immunoglobulin complex.

2. The peptide as set forth in claim 1, wherein:
the peptide comprises at least one selected from the group consisting of: a partial peptide of Protein A; a partial peptide of Protein G; a partial peptide of Protein H; a partial peptide of Protein M; a partial peptide of a rheumatoid factor; and a partial peptide of a complement.

3. The peptide as set forth in claim 2, wherein:
the partial peptide of Protein A, the partial peptide of Protein G, the partial peptide of Protein H, the partial peptide of Protein M, the partial peptide of the rheumatoid factor, and the partial peptide of the complement are respectively mutants thereof.

4. The peptide as set forth in claim 1, wherein:
the peptide is a partial peptide of Protein G or a mutant of the partial peptide.

5. The peptide as set forth in any one of claims 1 through 4, wherein:
the labeling agent comprises at least one selected from the group consisting of a radioactive substance, an enzyme, a coenzyme, a luminescence substance, a fluorescence substance, and a fluorescent protein.

6. The peptide as set forth in any one of claims 1 through 5, wherein:
the labeling agent comprises at least one selected from the group consisting of 35S radioactive labeling methionine, horseradish peroxidase, alkaline phosphatase, biotin, an acridinium derivative, fluorescein, and green fluorescent protein.

7. The peptide as set forth in any one of claims 1 through 6, wherein:
the peptide is bonded with the labeling agent through covalent bonding.

8. A method for detecting or assaying an immunoglobulin through use of a peptide as set forth in any one of claims 1 through 7.

9. The method as set forth in claim 8, said method comprising the steps of:
(a) causing a solution containing an immunoglobulin to be in contact with a solid phase, so that the immunoglobulin is directly or indirectly bound to or adsorbed onto the solid phase;
(b) causing the immunoglobulin to be in contact with a peptide as set forth in any one of claims 1 through 7, so that the immunoglobulin is bound to the peptide; and
(c) assaying an amount of the peptide bound to the immunoglobulin.

10. The method as set forth in claim 9, wherein:
an antigen which binds to the immunoglobulin is immobilized to the solid phase in advance.

11. A kit for detecting or assaying an immunoglobulin, comprising a peptide as set forth in any one of claims 1 through 7.
